Europäisches Patentamt

European Patent Office

Office européen des brevets

(11) Publication number: **0 211 260 B1**

# EUROPEAN PATENT SPECIFICATION

(45) Date of publication of patent specification: 06.03.91

(51) Int. Cl.⁵: **C12N 15/58**, C07H 21/04, C12N 5/00, C12N 9/48

(21) Application number: 86109385.4

(22) Date of filing: 09.07.86

(54) Chromosomal DNA sequence, expression vector for human tissue plasminogen activating factor, cultured cells transfected with same and method of producing said activating factor.

(30) Priority: 10.07.85 JP 152810/85
31.01.86 JP 20469/86
26.04.86 JP 97481/86

(43) Date of publication of application:
25.02.87 Bulletin 87/09

(45) Publication of the grant of the patent:
06.03.91 Bulletin 91/10

(84) Designated Contracting States:
BE CH DE FR GB IT LI NL SE

(56) References cited:
EP-A- 0 093 619
EP-A- 0 117 059

(73) Proprietor: KANEGAFUCHI KAGAKU KOGYO KABUSHIKI KAISHA
2-4 Nakanoshima 3-chome
Kita-ku Osaka-shi Osaka-fu(JP)

(72) Inventor: Kakutani, Tetsu
Manhaimu Kakogawa No. 626 835-1, Befu
Befu-cho
Kakogawa-shi Hyogo-ken(JP)
Inventor: Matsumoto, Keiji
20-16-204, Tendo-cho
Nishinomiya-shi Hyogo-ken(JP)
Inventor: Yahara, Hitoshi
830-161, Ikeda Onowe-cho
Kakogawa-shi Hyogo-ken(JP)
Inventor: Maruyama, Hiroyuki
2-63, Okihama-cho Takasago-cho
Takasago-shi Hyogo-ken(JP)
Inventor: Kawaharada, Hajime
2183-4, Shinzaike Hiraoka-cho
Kakogawa-shi Hyogo-ken(JP)
Inventor: Watanabe, Kiyoshi
15-41, 5-chome, Matsugaoka
Akashi-shi Hyogo-ken(JP)

(74) Representative: **Vossius & Partner**
**Siebertstrasse 4 P.O. Box 86 07 67**
**W-8000 München 86(DE)**

# EP 0 211 260 B1

## Description

This invention relates to a DNA sequence comprising a chromosomal DNA sequence coding for human tissue plasminogen activating factor (hereinafter abbreviated as "TPA") and a promoter region DNA sequence capable of functioning in animal cells as joined together, to cells capable of producing TPA as a result of transfection with said DNA sequence and to a method of producing TPA using said cells. The invention further relates to an expression vector for TPA which has an amplifiable gene DNA sequence, to cultured animal cells transfected with said expression vector and to a method of producing TPA using said cells. Furthermore, the invention relates to cultured animal cells transfected with an expression vector for TPA and a DNA having an amplifiable gene and to a method of producing TPA using said cells.

Plasminogen activating factor converts plasminogen occurring in the blood to plasmin which has fibrinolytic activity. It is used as a therapeutic agent for various types of thrombosis.

A known method of producing TPA comprises cultivating a cell line capable of producing this factor and purifying the same from the culture medium [D.C. Bijken and D. Collen (1981), Journal of Biological Chemistry, vol. 256, p. 7035]. This method is greatly dependent on the TPA production capacity of the cell line and can hardly enable large-scale TPA production. The cDNA (complementary DNA) for TPA has been cloned and the production of a TPA-like protein in Escherichia coli has become possible [D. Pennica et al. (1983), Nature, vol. 301, p. 214]. However, since the mechanisms of protein synthesis in microorganisms are more or less different from those in animal cells, such microbially produced TPA-like protein often has an amino terminus differing from that of the naturally occurring TPA. Moreover, the microbially produced TPA-like protein does not contain a sugar moiety whereas the naturally occurring TPA is glycosylated. Such TPA-like protein produced through the protein synthesis system of a microorganism is quite different as a substance, from the naturally occurring TPA and, for its long-term or repeated use as a therapeutic agent, such problems as decrease in potency and allergic reactions are expected to be encountered.

It was also attempted to produce TPA by transfecting CHO (chinese hamster ovary) cells with a plasmid containing a DNA sequence composed of the DNA sequence for TPA and the early promoter of SV40 as joined together and a DNA sequence coding for dihydrofolate reductase and further selecting those cells in which the gene has been amplified using a methotrexate-containing medium (Japanese Kokai Tokkyo Koho No. 59-42321). However, the DNA for TPA as used in this production method was cDNA.

In a further case which is known, the chromosomal DNA for human TPA was cloned and used for transfection of murine cells and TPA-producing cells were selected Since, however, the promoter activity of the TPA gene was relatively low, the TPA production ability of the transfeced cells was limited in this method [M. J. Brown et al. (1985), Gene, vol. 33, p. 279].

It is an object of the invention to provide a novel chromosomal DNA sequence.

Another object of the invention is to provide a DNA sequence comprising a chromosomal DNA sequence for TPA and the DNA sequence of a promoter region which is capable of functioning in animal cells as joined together, cells capable of producing TPA'as a result of their transfection with a DNA having said DNA sequence, and a method of producing TPA using said cells.

A further object of the invention is to provide an expression vector for TPA which comprises the DNA se quence of an amplifiable gene, transfected cells derived from cultured animal cells by using said expression vector and a method of producing TPA using said cells.

A still further object of the invention is to provide cultured animal cells transfected with a TPA expression vector and a DNA containing an amplifiable gene and a method of producing TPA using said cells.

In the accompanying drawings,

Fig. 1 shows a gene region coding for TPA and TPA gene fragments cloned in the recombinant phages λPABg15.7, λPAEcoll, λPASst9 and λPABg16.0;

Fig. 2 shows the restriction enzyme cleavage map each of pPASst9 and pPASst9-S;

Fig. 3-(a) shows the structure of the plasmid pPAEcoll, Fig. 3-(b) the structure of the DNA structure of the phage M13BA137, Fig. 3-(c) the structure of the plasmid pPASst9, Fig. 3-(d) the structure of the plasmid pPAIII and Fig. 3-(e) the structure of pPAHin3.3;

Fig. 4 shows the construction scheme for the plasmids pSVeBall•HindIII and pSVeSalI;

Fig. 5 shows the construction scheme for the plasmid pPAeI-3;

Fig. 6 shows the construction scheme for the plasmid pPAII-2;

Fig. 7 shows the construction scheme for the plasmid pPAeIV-2;

Fig. 8 shows the construction scheme for the plasmid pSVePA-I;

Fig. 9-(a) shows the construction scheme for the plasmid pPAIV-I and Fig. 9-(b) the construction scheme for the plasmid pSV3LPA;

3

Fig. 10 shows the construction scheme for the plasmid pSVpTKPA;

Fig. 11 shows the construction scheme for the plasmid pSVePA3; and

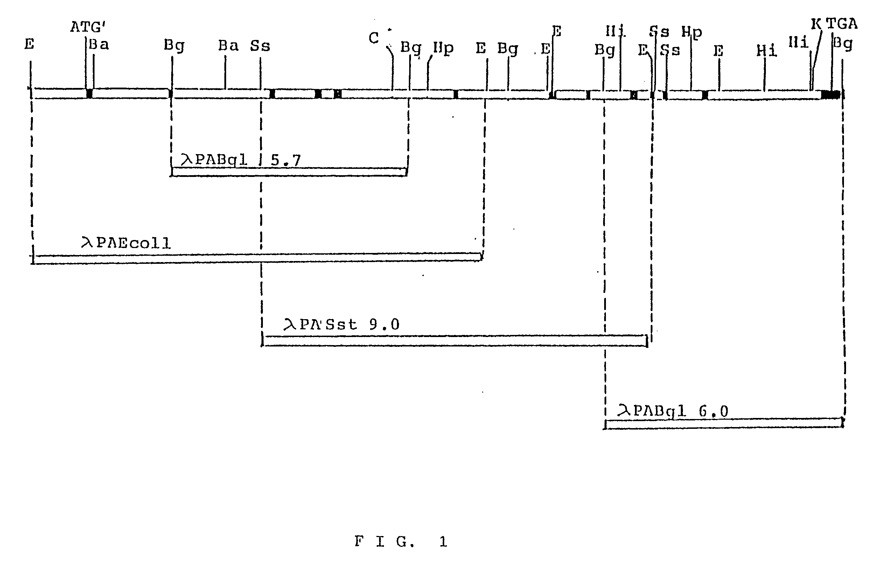

In Fig. 1, the black areas each represents an exon and there are shown, from left to right, the 2nd to the 14th exon, the numbering being according to Ny et al. [Ny et al. (1984), Proceedings of the National Academy of Sciences of the USA, vol. 81, p. 53].

Throughout Figs. 1-9 and Fig. 11, E, Ba, Bg, Ss, C, Hp, Hi, K, Xb, Sc, Sa, Ac, P, X and Ball indicate the recognition sites by the restriction enzymes Eco RI, Bam HI, Bgl II, Sst I ( Sac I), Cla I, Hpa I, Hind III, Kpn I, Xba I, Sca I, Sal I, Acc I, Pvu II, Kpn I and Bal I, respectively. TPA, Amp, SVe, ori., Ecogpt, T-ag., pA-(polyA), TK, dhfr and pTX show the TPA gene or part thereof, the ampicillin resistance gene, the early gene promoter region of SV40 which contains the DNA replication origin, the plasmid replication origin, the Ecogpt gene, the T antigen gene of SV40, a poly(A) addition signal of SV40, the thymidine kinase gene of HSV-1, the dihydrofolate reductase gene and the promoter region of the thymidine kinase gene, respectively.

To solve the afore-mentioned problems concerned with the product TPA or involved in the production of TPA, the present inventors conducted investigations from the following viewpoints using nuclear DNAs.

It is known that many proteins produced in higher animals are each encoded on a nuclear DNA sequence by a gene interruptedly divided into a certain number of segments. The DNA sequence segments coding for the sequence of a mature messenger RNA (mRNA) are called exons and the intervening sequences are called introns. The TPA gene is composed of at least 13 exons and 12 introns, with a sequence adjacent to the 5' end and a sequence adjacent to the 3' end.

The removal of such introns from the nascent RNA synthesized is called splicing. The process of splicing is assumed to be necessary for the accumulation of stable mRNAs or for the transfer of mRNAs from the nucleus to the cytoplasm. The present inventors assumed that mRNAs after splicing are more stable than mRNAs just after transcription from cDNA sequences and can be accumulated in high concentrations in the cytoplasm.

Splicing of introns at correct position is essential for the expression of normal and functioning proteins. A report, however, described abnormal splicing in the case of introduction into COS cells of the insulin gene and the promoter region of simian virus 40 (SV40) as joined together [0. Laub et al. (1983), Journal of Biological Chemistry, vol. 258, p. 6043].

Therefore, when the TPA gene containing introns is introduced into cultured animal cells, it is necessary for the production of TPA that normal splicing shold take place. The present inventors have found that the TPA-encoding nuclear DNA isolated from the human nuclear DNAs involves two types, one in which an Sma I recognition site is present between the 8th and the 9th exon and another which is free of such site, that the Sma I site-free type lacks an about 0.3 kb region in the neighborhood of the Sma I site as compared with the Sma I site-containing type and that even such Sma I site-free chromosomal DNA sequence can be spliced in a normal manner to cause secretion of a significant amount of TPA. These findings have now led to the present invention.

TPA is a glycoprotein. The structure of the sugar chain of TPA remains unknown in many aspects and whether TPA species produced in cells other than human cells differ in sugar chain structure and in antigenicity from the TPA produced in human-derived cells also remains unknown. It is considered, however, that the TPA produced in human cells could be undistinguishable from the naturally occurring TPA species but would be higher in safety than those TPA species produced in cells other than human cells. It is expected that TPA products secreted by human cells using an adequate medium will contain no other substances than those which are essentially human components or, in other words, those substances which are present in the human blood, and that such TPA products therefore will have improved safety features.

Many kinds of cells have the property of attaching themselves to the wall of a vessel or tank. Even when cultivated with forced stirring of the culture medium, cell having such property can hardly grow as separate cells but show a tendency toward aggregation. Since cell aggregates are not uniform in size, the cell density in the medium lacks uniformity. Furthermore, the control of stirring is difficult; gentle stir ring tends to allow adherence of cells to the vessel wall while vigorous stirring may possibly damage cells. The present inventors have succeeded in isolating several TPA-producing cell lines via transfection of blood cell-derived established cell lines capable by nature of growing as separate individual cells without attachment to the vessel wall and without marked aggregation. These TPA-producing cell lines can be cultivated very easily by suspension culture and thus can be cultivated on a large scale.

When a gene is introduced into a cell, the gene introduced is in some instances incorporated stably into a chromosomal DNA of the host. The locus on the chromosome where gene incorporation takes place is apparently at random and the number of copies of the DNA incorporated is also variable. When a TPA expression vector is introduced into cells, the locus of incorporation and the copy number vary from cell to cell and accordingly the TPA production varies from cell to cell. Therefore, cells varying in productivity can be obtained by cell cloning. The yield of TPA is considered to be correlated with the number of copies of the TPA gene and it is expected that a cell with an increased number of TPA genes will have an increased TPA production capacity.

The present inventors have succeeded in cultivating cell lines having high TPA producitivity by introducing a TPA expression vector containing an amplifiable gene into cultured animal cells or by introducing a TPA expression vector and an amplifiable gene into such cells, followed, in each case, by isolation of cells under selectable conditions. In that case, the TPA gene used for constructing the TPA expression vector was a human TPA gene derived from a normal human chromosomal DNA and deficient in the Sma I site between the 8th and the 9th exon.

These cell lines capable of producing TPA in high yields were cells capable of producing TPA even in a serum-free medium. The use of a serum-free medium in the production of TPA not only facilitates the purification of TPA but also prevents the possible product contamination with serum components.

Thus the present invention has made it possible to provide a very highly safe, natural type TPA species in large quantities.

As a result of gene cloning, it has been confirmed that the gene region coding for the amino acid sequence of TPA has a length of about 18 kilobases (kb). Ny et al. have shown that the gene is composed of at least 14 exons and 13 introns [Ny et al. (1984), Proc. Natl. Acad. Sci. USA, vol. 81, p. 5355].

The chromosomal DNA sequence coding for TPA is a DNA sequence containing the sequence from the codon ATG for the TPA translation initiation amino acid methionine to the termination codon TGA.

The TPA-encoding chromosomal DNA is cloned from a human DNA. The human DNA is prepared, for example, by using cultured human leucocytes or tissue and following the procedure of Blin et al. [N. Olin et al. (1976), Nucleic Acids Res., vol. 3, p. 2303]. As the vector for cloning the TPA gene, there may be used a λ phage vector, typically Charon 28, a plasmid vector, typically pBR322, or a cosmid, typically pHC79. A gene manipulation technique applicable to the case where a λ phage is used as the vector is now described by way of example. A human macromolecular DNA is cleaved with adequate restriction enzymes and fragments are inserted into a λ phage vector for a substitutable region thereof, to give recombinant DNAs. Then, infectious phage particles are prepared by the technique of in vitro packaging. A culture plate is inoculated with the particles and host Escherichia coli cells to thereby cause formation of recombinant phage plaques [L. Enquist et al. (1979), Methods in Enzymology, vol. 68, p. 281; B. Horn (1979), Methods in Enzymology, vol. 68, p. 299]. For detecting plaques due to recombinant phages having a DNA fragment coding for TPA, the technique of plaque hybridization [S. L. C. Woo (1979), Methods in Enzymology, vol. 68, p. 389; J. W. Szostak et al. (1979), Methods in Enzymology, vol. 68, p. 419] using a cDNA coding for TPA or a synthetic DNA having the DNA sequence of part of the TPA gene as a probe can be employed. The recombinant phage having the TPA gene can be prepared in large quantities by recovering the phage from a plaque selected by plaque hybridization and cultivating the same with the host Escherichia coli. The recombinant phage DNA can be prepared by the phenol extraction; see T. Maniatis et al. (1982), Molecular Cloning, A Laboratory Manual, Cold Spring Harbor Laboratory], for instance.

In case the TPA gene has been cloned in a plurality of DNA fragments, the complete TPA gene can be reconstructed from the respective fragments.

In use for the DNA introduction into cultured animal cells are the calcium phosphate method [M. Wigler et al. (1977), Cell, vol. 11, p. 223], the microinjection method [W. F. Anderson et al. (1980), proc. Natl. Acad. Sci. USA, vol. 77, p. 5399], the liposome method, the DEAE-dextran method and the cell fusion method [W. Schoffner et al. (1980), Proc. Natl. Acad. Sci. USA, vol. 77, p. 2163], for instance, with varying transfection efficiency. As the DNA material for use in carrying out the calcium phosphate method, there may also be used a microorganism, such as Escherichia coli, a phage and so on which bear the desired DNA sequence. For the cell fusion method, the protoplast of a microorganism carrying the desired DNA sequence as a plasmid.

The present inventors have found that the promoter region sequence functioning in cultured animal cells, namely of a gene other than the TPA gene, when joined to the chromosomal DNA sequence for TPA on the 5' end thereof and introduced into non-TPA-producing cells, can transfect said cells, giving cells highly productive of TPA. In this case, the mRNA for TPA is synthesized under the control of the connected promoter region and, in case the promoter region joined is the promoter region for a constitutive protein, the mRNA for TPA is constantly synthesized in the cells and said cells thus become cells for constitutive TPA production. In case the promoter region connected is for an inducible protein, the transfected cells produce TPA as an inducible protein.

The early promoter of SV40 is known as one of the promoters functioning in cultured animal cells. This promoter is contained in an about 340 base pair (bp) HindIII-Pvu II fragment of the SV40 DNA. This DNA fragment also has an activity as the late gene promoter of SV40 in the reverse direction.

Transcription activity from the late gene promoter of SV40 is generally enhanced in the presence of T-antigen of SV40. Therefore, for the cell wherein TPA gene linked to the late gene promoter is introduced, the cell which can express T-antigen gene is preferable employed. The cell wherein T-antigen gene is expressed can be prepared by introducing T-antigen-coding gene into the cell. When the cultured cell is transfected with a DNA sequence wherein TPA gene linked to T-antigen DNA sequence of SV40 and to the late promoter of SV40 is present on one and the same sequence, a cell line which shows high TPA expression will be obtained in many kinds of the established cell.

The type I thymidine kinase promoter of herpes simplex virus (HSV) is also a constitutive promoter like the early gene promoter of SV40. The structure of said promoter region has been shown by Wagner et al. [M. J. Wagner et al. (1981), Proc. Natl. Acad. Sci. USA, vol. 79, p.1441].

Said "promoter region functioning" means the sequence of a promoter region which contains the mRNA synthesis startpoint but does not contain the codon for the first amino acid methionine of the protein which is under the regulation by the promoter.

The construction of a DNA (TPA expression vector) by connection of a promoter region sequence to the TPA-encoding chromosomal DNA sequence as performed from the above viewpoint will be described later in a working example.

For selection of the cell in which the desired gene introduced can be expressed stably, a DNA sequence having, on one and the same DNA sequence, a sequence comprising a promoter sequence and the TPA gene joined together on one hand and, on the other, a selectable marker gene is suited. Usable as the selectable marker gene applicable in animal cells are the genes Ecogpt [R. C. Mulligan et al. (1980), Science, vol. 209, p. 1422], neo [P. J. Southern et al. (1982) , Journal of Molecular and Applied Genetics, vol. 1, p. 327] and dhfr [M. Wigler et al. (1980), Proc. Natl. Acad. Sci. USA, vol. 77, p. 3567], among others. For preparing such DNA sequence in large quantities, it is desirable that such DNA sequence is a plasmid or phage DNA which can replicate in large quantities in Escherichia coli . The plasmids pSVePA-1, pSV3LPA and pSVpTKPA are plasmids suited for the above purposes. They are characterized in that a DNA replication origin (ori) which enables their replication in Escherichia coli , a selectable marker gene (ampicillin resistance gene), a selectable marker gene to serve in cultured animal cells (Ecogpt), and the TPA-encoding chromosomal DNA sequence with a promoter as joined thereto are present on one and the same DNA sequence.

For the cells with the TPA-encoding chromosomal DNA sequence and the promoter region joined together and introduced thereinto to produce TPA, it is necessary that said DNA sequence should match with the intrinsic RNA synthesizing system of the cells, the maturation of RNA, the protein synthesizing system, the maturation and secretion of protein and other functions. While mRNA synthesis takes place on the DNA introduced, the addition of the cap structure to the 5' end of mRNA, splicing at right positions and polyadenylation of the 3' end are necessary. For the expression of active TPA, it is necessary that normal higher structures of the TPA peptide synthesized should be built up and maintained and further that cleavage of the signal peptide and secretion of TPA from cells should be conducted accurately. While, by way of trial, the present inventors used, as the cultured animal cells, those hamster-, monkey-, mouse-, and human-derived cells that are available from the American Type Culture Collection (ATCC), the TPA production method disclosed herein can be applied to the production of active TPA at least in vertebrate-derived cultured cells, fused cells, normal and mutant cells, virus-transfected cells, etc.

At present, the structure of the sugar chain of TPA remains unknown in many aspects. For instance, it is unknown whether the TPA species produced in hamster cells differs in sugar chain structure and antigenicity from the TPA species produced in human cells. At any rate, however, the TPA species produced in human cells is a naturally occurring one and will avoid such problems as allergic reactions and shock in long-term administration thereof as a therapeutic agent. It is also expected that the use as the TPA-producing cells of those established cell line cells that have been derived from human cells by transfection with SV40 might increase the safety of the product because proper measures can be taken as compared with the case where a cell line which has become carcinomatous or established for an unknown cause is used. The human cell line WI-26 VA4 (CCL-95.1) is known as an SV40-transfected one.

It has been found that cells of a blood cell-derived established cell line can be transfected with a DNA constructed so as to enable TPA expression. Said blood cell-derived established cell line, when it is of the mammalian origin, means a cell line established from among lymphoblasts, myeloblasts, monoblasts and erythroblasts respectively derived from stem cells, and cells differentiated or derived therefrom. When grown in a usual medium, cells of a blood cell-derived established cell line morphologically take a spherical shape in the main, hence are suited for suspension culture. Some of such established cell line cells produce globulin and are therefore considered to be highly capable of synthesizing and secreting proteins.

The use of such cells as the hosts is advantageous from the TPA production viewpoint. While the established cell lines used by way of trial were MOPC-11 (mouse blastocytoma: CCL-130) and MPC-11 (mouse myeloma: CCL-167), both acquired from the American Type Culture Collection, the TPA production method disclosed herein can be applied to the production of active TPA at least in mammalian (inclusive of human) blood cell-derived established cell lines, hybridoma mutants and virus-transfected cells derived therefrom, and so on.

The TPA production by TPA expression vector-transfected cells is considered to be correlated with the number of TPA gene copies contained in the transfected cells. Cells carrying TPA gene copies in great numbers and thus highly productive of TPA could be isolated, following introduction of a TPA expression vector having an amplifiable gene, by single cell isolation or cell selection under culture conditions such that cells with the amplifiable gene amplified therein can grow selectively.

Usable in constructing TPA expression vectors having such an amplifiable gene are such amplifiable genes as the dihydrofolate reductase gene, aspartate transcarbamylase gene and metallothionein gene. Other amplifiable genes [G. R. Stark and G.M. Wahl (1984), Annual Review of Biochemistry, vol. 53, p. 447] can also be employed.

From among cells transfected with a TPA expression vector having the dihydrofolate reductase gene, those cells with the dihydrofolate reductase gene amplified therein can be selected, after transfection, by using a medium containing methotrexate in a concentration of not less than 1 nM. In the cells thus selected, not only the dihydrofolate reductase gene but also the TPA gene can often be found amplified. The present inventors also could obtain highly productive cell lines by selective isolation, after transfection, of transfected cells using a medium containing methotrexate (Mtx) in a concentration of not less than 1 nM. Similarly, cells with the metallothionein gene amplified therein can be isolated by using a heavy metal ion and cells with the aspartate transcarbamylase gene amplified therein by using N-(phophonacetyl)-L-aspartate (PALA). When the dihydrofolate reductase gene (dhfr) is used as the amplifiable gene, it is advantageous to use a dhfr cell line as the host since the isolation by means of Mtx of a cell line with the gene amplified therein can be conducted with ease.

Cells having the same properties as the cells transfected with a TPA expression vector having an amplifiable gene can also be obtained by cotransfection with a DNA having an amplifiable gene and a TPA expression vector which has no such amplifiable gene. The TPA gene in cells obtained by cotransfection is amplified with the amplifiable gene.

It has been found that when the TPA-encoding chromosomal DNA sequence with a promoter joined thereto is introduced into cultured animal cells by the calcium phosphate method or cell fusion method, for instance, cells which have thus become productive of TPA can produce TPA not only in a serum-containing medium, which is common in cell culture, but also in a serum-free medium. By using a serum-free medium in the production of TPA, TPA recovery from the medium and purification thereof can be further facili tated. For the TPA recovery from medium and purification of TPA, known processes can be applied. Thus, for instance, TPA can be recovered and purified by chromatography or electrophoresis using chelating Sepharose, lysine-Sepharose, ConA-Sepharose, ion exchangers, Sephadex gel, etc.

The activity of TPA can be measured by the plasminogen-containing fibrin plate method [M. Mackie et al. (1981), British Journal of Haematology, vol. 47, p. 77] or the method based on the decomposition of S 2251, a synthetic coloring substrate for plasmin [R. A. Allen and D. S. Pepper (1981), Thrombos. Haemostas., vol. 45, p. 43]. Certain kinds of cells are capable of producing urokinase. The production of TPA by such cells can be measured by treating samples with anti-urokinase antibody in advance. The plasminogen activating factor produced by the transfected cells can be identified by zymography for molecular weight estimation and the neutralization test with anti-TPA antibody.

The following examples are further illustrative of the present invention. The experiments described herein were conducted in accordance with the "Guidelines for Recombinant DNA Experiments" established by the Prime Minister of Japan. For the details of the procedures followed in handling phages, plasmids, DNAs, various enzymes, Escherichia coli strains, etc., the following journal articles and monographs were consulted and are incorporated herein by reference:

1. Tanpakushitsu-Kakusan-Koso (Protein-Nucleic Acid-Enzyme), vol. 26, No. 4 (1981), an extra issue, Gene Manipulation, Kyoritsu Shuppan;

2. Idenshi Sosa Jikkenho (Experiments in Gene Manipulation, edited and partly written by Y. Takagi (1980), Kodansha;

3. Idenshi Sosa Manual (Manual of Gene Manipulation), edited and partly written by Y. Takagi (1982), Kodansha;

4. Molecular Cloning, A Laboratory Manual, edited by T. Maniatis et al. (1982), Cold Spring Harbor Laboratory;

5. Methods in Enzymology, vol. 68, edited by L. Grossman et Cal. (1980), Academic Press; and
6. Methods in Enzymology, vol. 65, edited by R. Wu (1979), Academic Press.

Example 1

Cloning of TPA gene

The human DNA used for the cloning of the gene was prepared as follows:

Blood was collected from a plurality of healthy humans and the buffy coat was collected. To the buffy coat was added about 10 volumes of 0.83% $NH_4Cl$ for hemolysis, followed by washing with Eagle's MEM medium to give leucocytes. The leucocytes ($10^{10}$ cells) were dissolved in 50 ml of a solution containing 0.5 M EDTA, 0.5% Sarcosyl and 100 μg/ml protease K at 50°C with shaking for 3 hours. After three repetitions of extraction with phenol, the aqueous layer was dialyzed against 20 mM Tris-HCl (pH 8.0), 10 mM EDTA, 10 mM NaCl. The dialyzate was treated with ribonuclease (100 μg/ml) at 37°C for 3.5 hours and, after extraction with phenol, further dialyzed against 20 mM Tris-HCl (pH 8.0), 1 mM EDTA, 10 mM NaCl, to give about 33 mg of macromolecular human DNA. Charon 28 and λgtwes•λB' were used as λ phage vectors. The phage DNAs were each cleaved with the restriction enzymes Bam HI, Eco RI or Sst I, as the case may be, and a fraction containing the left and right fragments (arms) of the phage DNA was collected by centrifugation on a sucrose density gradient, followed by recovery by precipitation with ethanol.

The TPA gene was partly cloned in four recombinant phage DNAs, as described later herein. The four DNA fragments shown in Fig. 1 are those recombinant phages λPABgl5.7, λPAEcoll, λPASst9.0 and λPABgl6.0 which contain the respective TPA gene fragments. In the figure, the black areas are exons, namely the 2nd to the 14th exon (from left to right), the exons being numbered according to Ny et al. [Ny et al. (1984), proc. Natl. Acad. Sci. USA, vol. 81, p. 5355].

The phage clone λPASst9-S obtained simultaneously with λPASst9.0 has an about 9 kb long Sst I fragment of the TPA gene, as in the case of λPASst9.0.

The about 9 kb Sst I fragment of TPA as contained in the λPASst9.0 or λPASst9-S DNA was subcloned in the plasmid PUC12 (obtained from Boehringer Mannheim) to give pPASst9 or pPASst9-S, respectively.

a) Comparison between pPASst9 and pPASst9-S

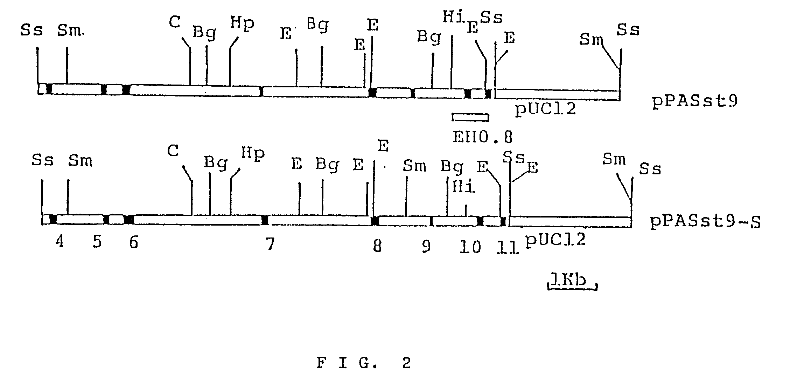

Analysis using restriction enzymes and base sequence determination of the TPA gene contained in pPASst9 and that contained in pPASst9-S have shown that there is a difference between both the DNAs with respect to an Sma I site, as shown in Fig. 2. Thus, it has been revealed that the Sma I site occurring in pPASst9-S between the 8th and the 9th exon is missing in pPASst9, which is also lacking in an about 0.3 kb segment inclusive of said site. The pPASst9 and pPASst9-S DNAs were respectively cleaved with the restriction enzymes Sst I and Sma I and the lengths of the resultant fragments were measured by agarose gel electrophoresis. As a result, pPASst9 gave an about 0.4 kb fragment and an about 8.5 kb fragment in addition to an about 2.7 kb fragement derived from the plasmid pUCl2 whereas pPASst9-S gave an about 0.4 kb fragment, an about 6.4 kb fragment and an about 2 kb fragment in addition to said pUCl2-derived, about 2.7 kb fragment. Then, Southern hybridization was conducted using as the probe an about 0.8 kb Eco RI-Hind III fragment containing the 10th and the 11th exon and $^{32}$P-labeled by nick translation. From among the above-mentioned fragments, the 8.5 kb fragment was detected in the case of pPASst9 and the 2 kb fragment in the case of pPASst9-S.

The above facts indicate that the human TPA gene includes two types differing in the segment located between the 8th and the 9th exon.

b. Cloning of 5.7 kilobase Bgl II fragment

The human DNA was cleaved with the restriction enzyme Bgl II and about 4.5-7.5 kb DNA fragments were recovered by centrifugation on a sucrose density gradient. These fragments were ligated with Charon 28 between the arms formed by Bam HI cleavage, in the presence of T4 DNA ligase. Using the ligation product and Escherichia coli LE392 as the host, in vitro packaging was conducted by the method of Enquist and Sternberg [L. Enquist and N. Sternberg (1979), Methods in Enzymology, vol. 68, p. 281] to cause formation of recombinant phage plaques. Then, a recombinant phage clone having the TPA gene was selected by the technique of plaque hybridization [W. D. Benton and R. W. Davis (1977), Science, vol. 196, p. 180]. For use as the probes, four oligonucleotides each having a sequence occurring in the TPA gene, namely the oligonucleotides

```
            5'                    3'
            GTCCTCGTAGCACG

            TAGCTGATGCCCTG

            TCCACGTGCCCCTG

            ACTCTCCGCTGTGC
```

were synthesized by the phosphotriester method [K. Miyoshi et al. (1980), Nucleic Acids Res., vol. 8, p. 5507] and then labeled at the 5'-OH using [ϒ-$^{32}$P]ATP and T4 polynucleotide kinase.

From among about 320 thousand recombinant phage clones, there was obtained a phage clone hybridizable with all the four synthetic DNA probes, which was named λPABgl5.7.

A 5.7 kb Bgl II fragment was prepared from the λPABgl5.7 DNA. Determination of the base sequence by the MI3 method [J. Messing and J. Vieira (1982), Gene, vol. 19, p. 269] of an Sau 3AI cleavage fragment derived from this 5.7 kb Bgl II fragment revealed the presence of a sequence having part of the cDNA sequence for TPA [D. Pennica et al. (1983), Nature, vol. 301, p. 214], namely

```
TC TGC AGA GAT GAA AAA ACG CAG ATG ATA TAC CAG
le Cys Arg Asp Glu Lys Thr Gln Met Ile Tyr Gln

CAT CAG TCA TGG CTG CGC CCT GTG CTC AGA AGC AAC
His Gln Ser Trp Leu Arg Pro Val Leu Arg Ser Asn

GTG GAA TAT TGC TGG TGC AAC AGT GGC AGG GCA CAG
Val Glu Tyr Cys Trp Cys Asn Ser Gly Arg Ala Gln

CAC TCA GTG CCT GTC AAA A GTATGTACTGAGGCTGGGAG
His Ser Val Pro Val Lys

GTGCATGCCTGTGATC.
```

On the other hand, analysis using various restriction enzymes showed that λPABgl5.7 contains the 5.7 kb Bgl II fragment of the TPA gene DNA shown in Fig. 1.

c. Cloning of 11 kb Eco RI and 9 kb Sst I fragments

An about 350 bp DNA fragment resulting from cleavage of the 5.7 kb Bgl II fragment of the HuTPA gene DNA with Pst I and containing the 4th exon was labeled with $^{32}$p by nick translation and, using this as the probe, Southern hybridization was conducted with the human DNA after digestion with various restriction enzymes. As a result, it was found that said about 350 bp Pst I fragment is hybridizable with the about 11 kb EcoRI fragment and the 9 kb Sst I fragment.

The human DNA was cleaved with the restriction enzyme Eco RI and the about 11 kb DNA fragment was recovered by centrifugation on a sucrose density gradient. This fragment was ligated with the arms of Eco RI-cleaved λgtwes•λ$_B$ in the presence of T4 DNA ligase, followed by in vitro packaging for formation of recombinant phage plaques with Escherichia coli LE392 as the host. Then, plaque hybridization was performed using, as the probe, said about 350 bp Pst I fragment labeled with $^{32}$P by nick translation. From among about 400 thousand recombinant phage clones, three phage clones hybridizable with the probe were picked out. Restriction enzyme cleavage analysis revealed that these phage clones all contain the about 11 kb Eco RI fragment of the TPA gene DNA shown in Fig. 1. One of the phage clones was named λPAEcoII. Plaque hybridization and Southern hybridization using, as the probe, a synthetic DNA oligomer,

5' ATGGATGCAATGAAG 3'

which corresponds to a partial amino acid sequence of TPA beginning with the startpoint amino acid, namely Met-Asp-Ala-Met-Lys-, revealed that the λPAEcoII DNA contains the above DNA sequence. The 11

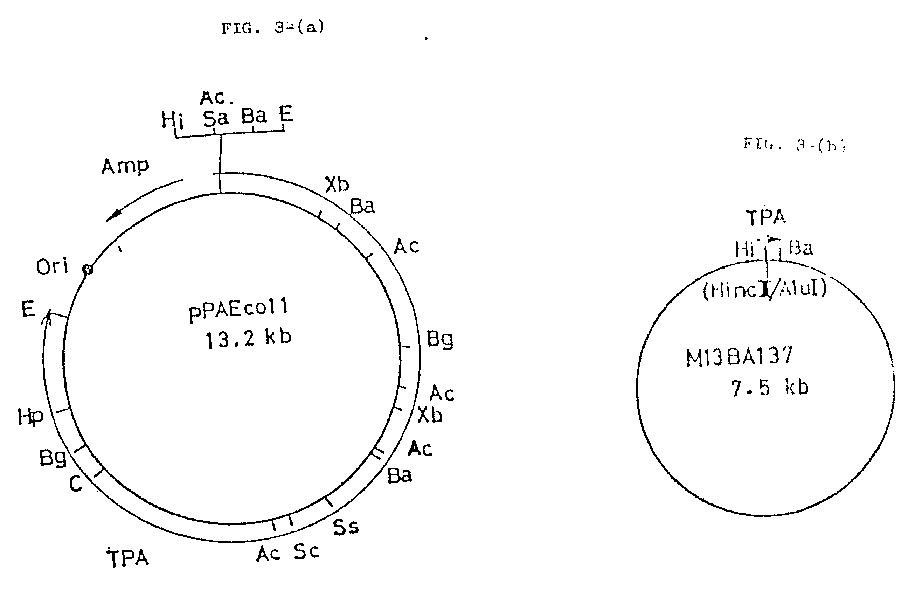

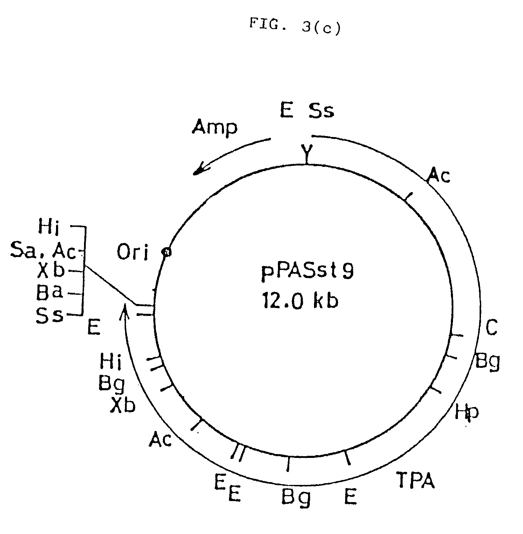

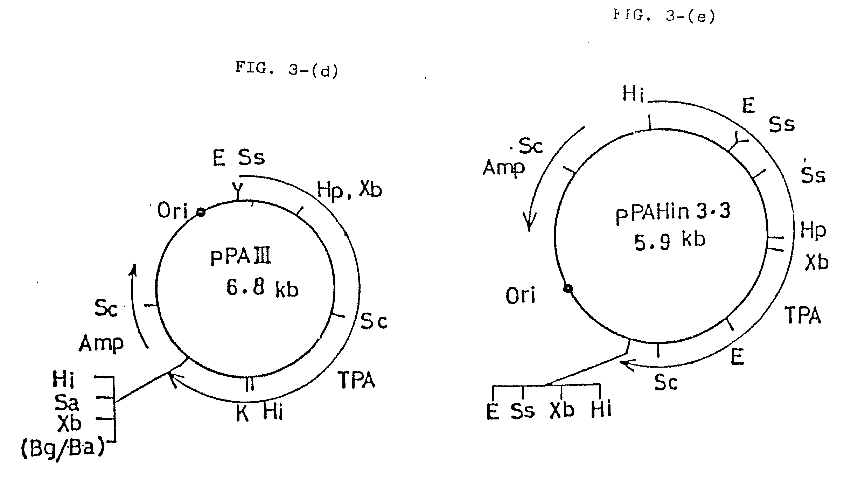

kb Eco RI fragment of HuTPA as contained in the λPAEcoII DNA was subcloned in the plasmid pUC9 [J. Vieira and J. Messing (1982), Gene, vol. 19, p. 259] at the Eco RI site thereof, to give pPAEcoII. The structure of pPAEcoII is shown in Fig. 3-(a) . Furthermore, pPAEcoII was cleaved with the restriction enzymes Eco RI and Bam HI. It was confirmed by Southern hybridization that the about 1.5 kb Eco I-Bam HI fragment thus obtained is hybridizable with the synthetic oligomer.

<div align="center">

**5' ATGGATGCAATGAAG 3'**

</div>

corresponding to the initial amino acid sequence of TPA following the initiation amino acid. The sequence of this DNA fragment was determined by the MI3 method. As a result, it was found that an Xba I-Bam HI fragment contained in the 1.5 kb Eco RI-Bam HI fragment codes for part of the signal peptide for TPA as follows:

<div align="center">

AGATGCATGTCTGGCGTTTCTGCCGCGGGGCTGGCACTGGAGGAAGC

ACAGGACAACATTGCGGCTTATCCTCCTCTGTTGCCATCATGGACCT

GCACCCCGTGATCAAGCTGTTTTTTTCTCTCCTTCCAG AATTTAAGG

GCTGTGAAGCAATC ATG GAT GCA ATG AAG AGA GGG CTC
                Met Asp Ala Met Lys Arg Gly Leu

TGT GTG CTG CTG CTG TGT GGA GCA GTC TTC GTT TCG
Cys Val Leu Leu Leu Cys Gly Ala Val Phe Val Ser


AGC CAG GTTGGTGTGCAGGATCC.
Ser Gln

</div>

The about 1.5 kb Eco RI-Bam HI fragment was further cleaved with the restriction enzyme Alu I, and the thus-obtained 137 bp Alu I-Bam I fragment containing the sequence coding for part of the signal peptide for TPA was inserted into the MI3 phage vector MI3mpII [J. Messing (1983), Methods in Enzymology, vol. 101, p. 20] at the Hinc II-Bam HI site. The structure of the thus constructed phage MI3Bal37 is shown in Fig. 3-(b).

Separately, the human DNA was cleaved with the restriction enzyme Sst I and the about 9 kb DNA fragment was recovered by sucrose density gradient centrifugation. Said fragment was ligated with the arms of Sst I-cleaved λgtwes•λ_B in the presence of T4 DNA ligase, followed by in vitro packaging. Using the technique of plaque hybridization, two clones hybridizable with said about 350 bp Pst I fragment were picked out from among about 500 thousand phage clones. Restriction enzyme cleavage analysis showed that these two phage clones contain the 9 kb Sst I fragment of the TPA gene DNA. One of them was named λpASst9.0. The 9 kb Sst I TPA DNA fragment contained in the λPASst9.0 DNA was subcloned into the plasmid pUCl2 to give a plasmid pPASst9. The structure of pPASst9 is shown in Fig. 3-(c).
d. Cloning of 6.0 kb Bgl II fragment

Cleavage of the 9 kb Sst I fragment of the TPA gene DNA with the restriction enzymes Hind III and Eco RI gives a 0.8 kb DNA fragment which contains the 10th exon. Digests of the human DNA with various restriction enzymes were subjected to Southern hybridization using said DNA fragment as the probe. An about 6.0 kb Bgl II-digested DNA fragment was found to hybridize with said probe.

The human DNA was cleaved with Bgl II and a fraction mainly containing an about 6.0 kb DNA fragment was recovered by sucrose density gradient centrifugation, and ligated with the arms of Bam HI-cleaved Charon 28 in the presence of T4 DNA ligase, followed by in vitro packaging. Using the technique of plaque hybridization, two phage clones hybridizable with the 0.8 kb Hind III-Eco RI fragment were picked out from among about 500 thousand phage clones obtained. Restriction enzyme cleavage analysis showed that these two phage clones contain the 6.0 kb Bgl II fragment of the TPA gene DNA shown in Fig. 1. One of them was named λPABgl6.0. The λPABgl6.0 DNA was found to contain a sequence corresponding to the C terminus of the TPA polypeptide as a result of plaque hybridization and Southern hybridization using, as the probes, the synthetic DNA oligomers

$$5' \; ACATGCGACCGTGA \; 3'$$

$$ACCAACTACCTAGA$$

which correspond to the amino acid sequences Thr-Asn-Tyr-Leu-Asp (515th to 519th amino acid) and Asn-Met-Arg-Pro-Op (524th to 527th amino acid), respectively, said amino acid sequences occurring near the C terminus of the polypeptide. The λPABgl6.0 DNA was cleaved with Sac I and Bgl II, and an about 4.3 kb Sac I-Bgl II fragment containing the 12th to 14th exons was separated and subcloned into pUCl2 at the Bam HI-Sac I site, to thereby construct a plasmid, pPAIII. The structure of pPAIII is shown in Fig. 3-(d). Furthermore, a plasmid, pPAHin3.3 was constructed by cleaving the λPABgl6.0 DNA with Hind III and subcloning an about 3.3 kb Hind III-cleaved DNA fragment containing the 10th to 13th exons into the plasmid pUCl2 at the Hind III site. The structure of ppAHin3.3 is shown in Fig. 3-(e).

Example 2

Construction of TPA expression vector pSVePA-1

An expression vector for TPA, pSVePA-1, was constructed via the steps a to e to be mentioned below.

a. Construction of pSVeBall.HindIII and pSVeSall

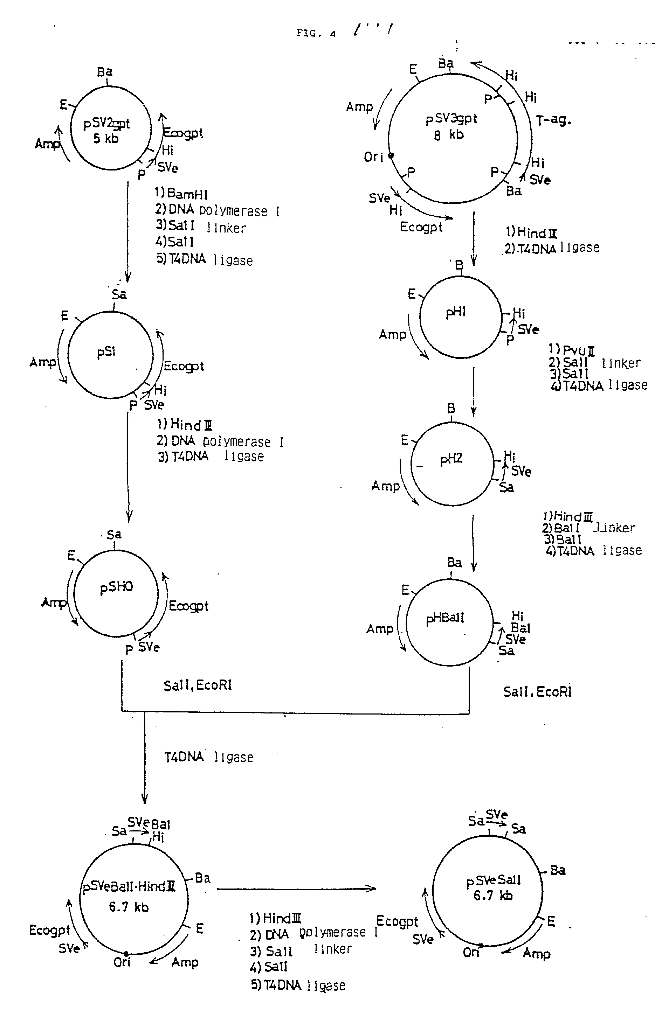

pSVeBall.HindIII and pSVeSall were constructed using pSV2gpt and pSV3gpt [R. C. Mulligan and p. Berg (1980), Science, vol. 209, p 1422] as the starting materials and following the scheme shown in Fig. 4. Thus, pSV3gpt was cleaved with the restriction enzyme Hind III, and the largest DNA fragment was made circular using T4 DNA ligase to give pH1. Then, the Pvu II site of PH1 was converted to an Sal I site using an Sall linker to give pH2. pH2 was cleaved with Hind III and further cleaved and end-repaired with DNA polymerase 1 (Klenow fragment), followed by incorporation of a Bal I site using a Bal I linker. Thus was constructed pHBall. Separately, pSV2gpt was cleaved with Bam HI, followed by conversion of the Bam HI site to an Sal I site using an Sall linker to thereby construct pS1. By further treating pS1 with Hind III and with DNA polymerase I (Klenow), a Hind III site-free plasmid, pSHO, was constructed. pHBall was cleaved with Sal I and Eco RI and the thus-obtained DNA fragment containing the promoter region of SV40 was ligated, in the presence of T4 DNA ligase, with a DNA fragment containing the ampicillin resistance gene as obtained by Sal I-Eco RI cleavage of pSHO. Thus was constructed pS VeBall.HindIII.

pSVeSall was constructed by Bal I cleavage of pSVeBall•HindIII, ligation with an unphosphorylated Sall linker in the presence of T4 DNA ligase and transformation of Escherichia coli.

The Sall linker and Ball linker used are d(pGGTCGACC) and d(pTTGGCCAA), respectively.

b. Construction of pPAel-3

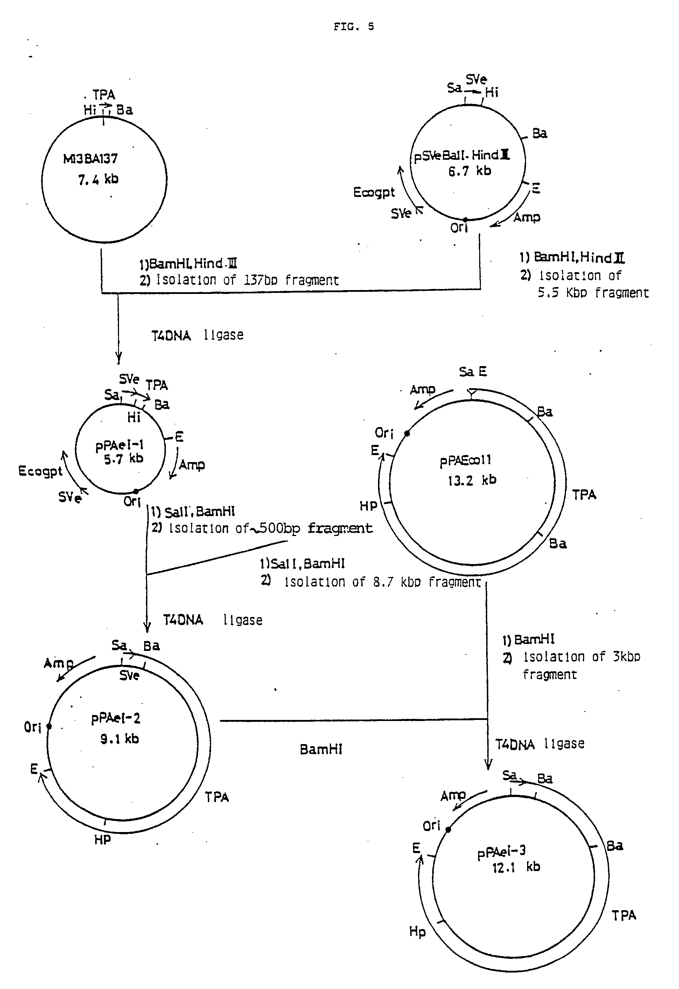

pPAel-3 was constructed according to the scheme shown in Fig. 5. By inserting an about 150 base Hind III-Bam HI fragment obtained from the double-strand M13Bal37 DNA and coding for the N-terminus of TPA into the plasmid pSVeBall•HindIII at the Hind III-Bam HI site thereof, there was constructed a plasmid, pPAel-1. Then, an about 500 bp Sal I-Bam HI fragment contained in pPAel-1 was inserted into pPAEco11 between the Sal I and Bam HI sites to give pPAel-2. An about 3 kb Bam HI-Bam HI fragment obtained from pPAEco11 was inserted into and ligated with pPAel-2 at the Bam HI site thereof. Thus was constructed pPAel-3.

c. Construction of pPAII-2

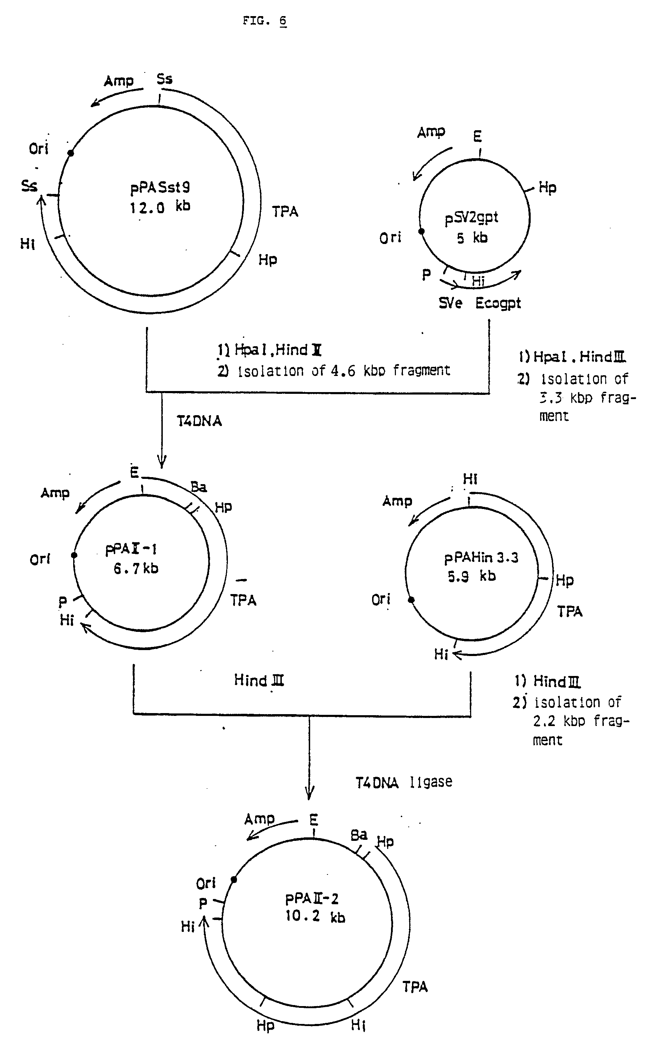

pPAII-2 was constructed in accordance with the scheme shown in Fig. 6. Thus, an about 4.6 kb Hpa I-Hind III fragment containing the 7th to 9th exons of the TPA gene as obtained from pPASst9 was inserted into pSV2gpt at the Hpa I-Hind III site thereof to thereby construct pPAII-1. Then, an about 3.3 kb Hind III-Hind III fragment excised from pPAHin3.3 was inserted into pPAII-1 at the Hind III site thereof to thereby construct pPAII-2.

d. Construction of pPAeIV-2

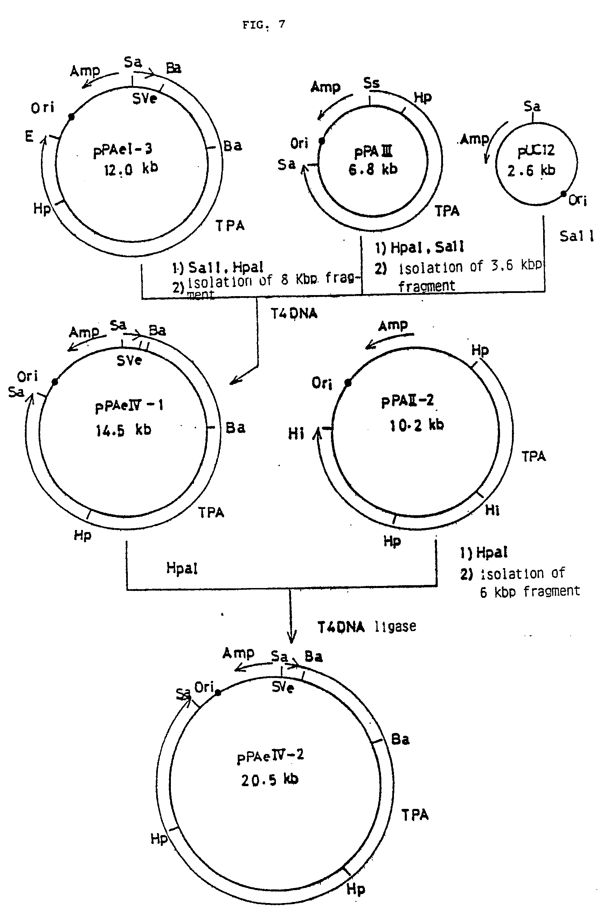

pPAeIV-2 was constructed in accordance with the scheme shown in Fig. 7. Thus, an about 8 kb Sal I-Hpa I fragment contained in pPAeI-3 and comprising the 2nd to 6th exons of the TPA gene and an about 3.6 kb Hpa I-Sal I fragment contained in pPAIII and comprising the 13th and 14th exons of the PTA gene were respectively isolated. These two DNA fragments were inserted into pUC12 at the Sal I site thereof to thereby construct pPAeIV-1. Then, an about 6.0 kb Hpa I-Hpa I fragment excised from pPAII-2 and containing the 7th to 12th exons of the TPA gene was inserted into pPAeIV-1 at the Hpa I site thereof. thus was constructed pPAeIV-2.

e. Construction of pSVePA-1

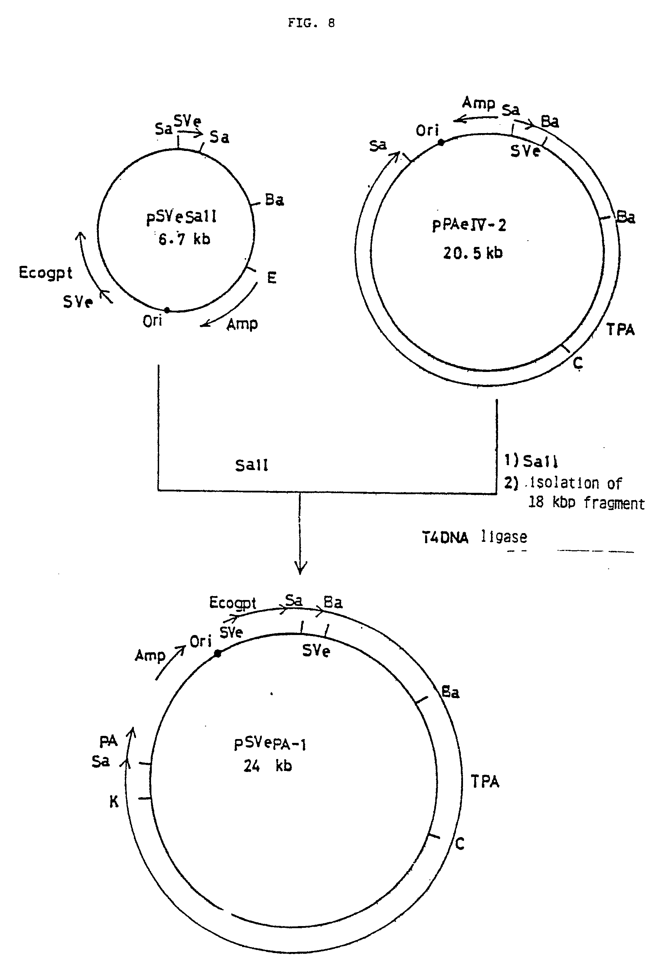

An expression vector, pSVePA-1, was constructed in accordance with the scheme shown in Fig. 8. Thus, an about 18 kb Sal I fragment excised from pPAeIV-2 and containing the promoter region of SV40 and the 2nd to 14th exons of the TPA gene was inserted into pSVeSalI at the Sal I site thereof. Thus was constructed pSVePA-1, with the promoter region containing ori of SV40 being joined to the TPA gene on the 5' side thereof and a sequence containing the polyadenylation signal of SV40 on the 3' side.

Example 3

Construction of TPA expression vector pSV3LPA

A TPA expression vector, pSV3LPA, was constructed via the steps a and b mentioned below.

a. Construction of pPAIV-2

pPAeI-3 was cleaved with Hind III and then made blunt-ended using DNA polymerase I (Klenow), followed by joining with an SalI linker and transformation of Escherichia coli K12 C600 r⁻ m⁻ . Thus was obtained pPAI4. The SalI linker used was d(GGTCGACC).

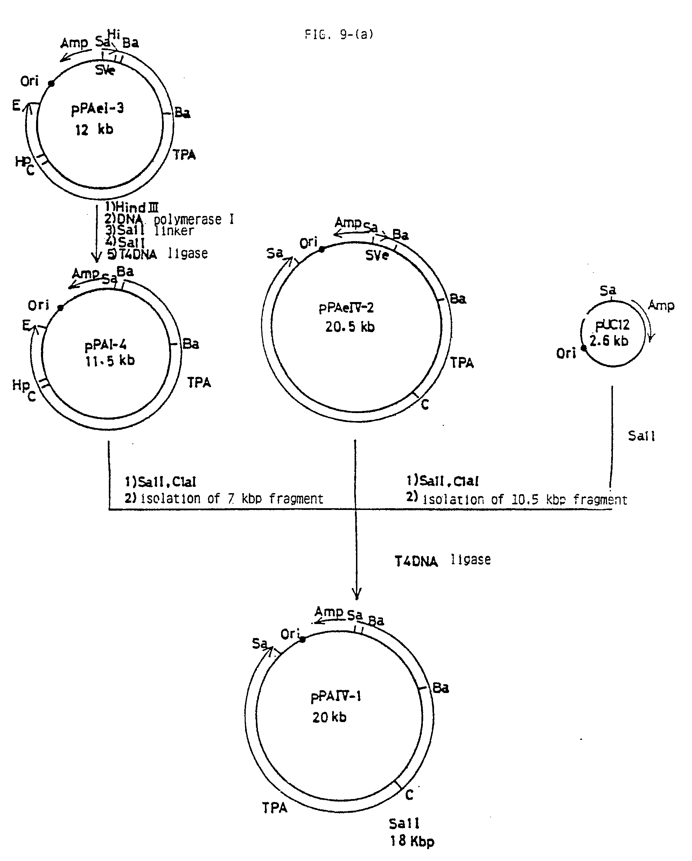

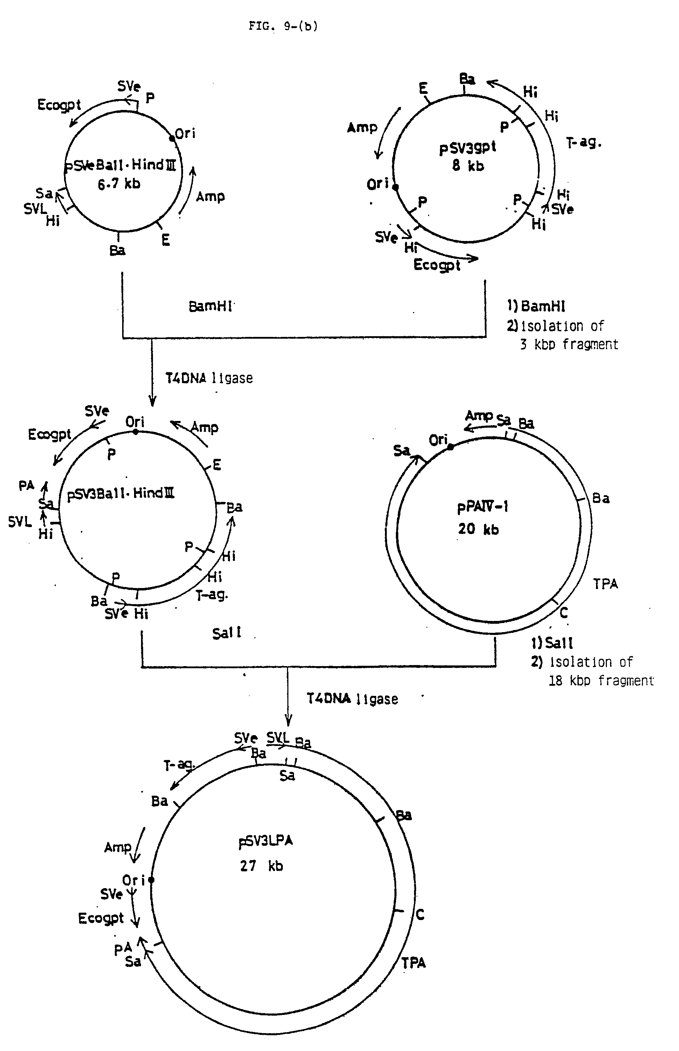

An about 7.0 kb Sal I-Cla I fragment containing the 2nd to 6th exons of the TPA gene was separated from pPAI-4 and an about 10.5 kb Sal I-Cla I fragment containing the 7th to 14th exons of the TPA gene from pPAeIV-2. These two DNA fragments were inserted into pUCl2 at the Sal I site thereof to construct pPAIV-1. The construction scheme for-pPAIV-I is shown in Fig. 9-(a).

b. Construction of pSV3LPA

An expression vector, pSV3LPA, was constructed in accordance with the scheme shown in Fig. 9-(b). Thus, a pSV3gpt-derived, about 3.0 kb long Bam HI fragment coding for T antigen of SV40 was inserted into pSVeBall•HindIII at the Bam HI site thereof to construct pSV3Ball•HindIII. Then, a ppAIV-I-derived, about 18 kb long Sal I-Sal I fragment covering the 2nd to 14th exons of the TPA gene was inserted into pSV3Ball•HindIII at the Sal I site thereof to give pSV3LPA.

Example 4

Construction of TPA expression vector pSVpTKPA

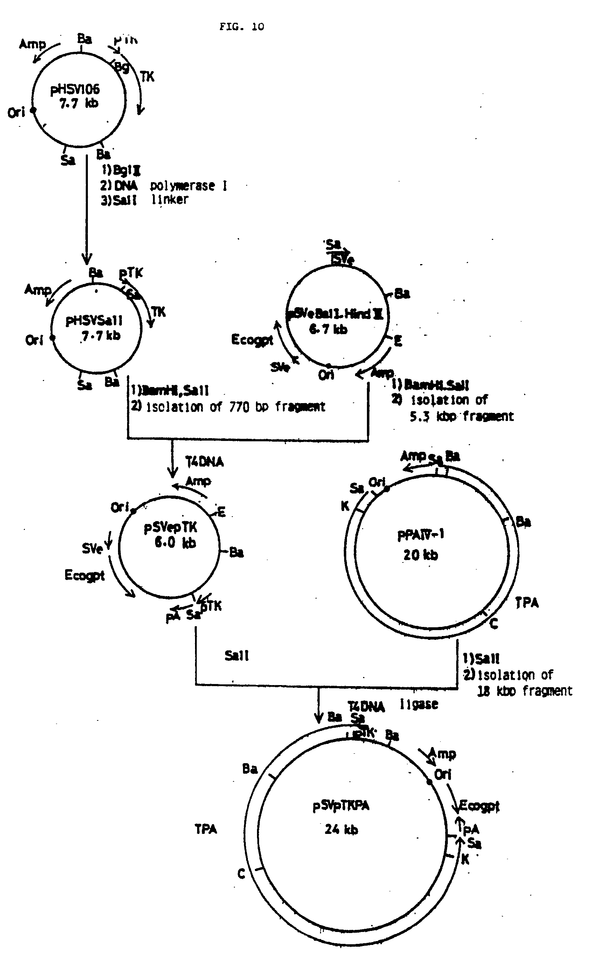

The construction scheme for a TPA expression vector, pSVpTKPA, is shown in Fig. 10. pHSV106 [S. L. McKnight and E. R. Gabis (1980), Nucleic Acids Res., vol. 8, p. 5931] has the thymidine kinase gene of herpes simplex virus. pHSV106 was cleaved with Bgl II and rendered blunt-ended with DNA polymerase I (Klenow), followed by conversion of the Bgl II site to an SalI site using an unphosphorylated SalI linker to give pHSVSalI. An about 770 bp Bam HI-Sal I fragment containing the thymidine kinase gene promoter region was excised from pSVHSalI and inserted into pSVeBall•HindIII between the Bam HI and Sal I sites thereof, whereby pSVpTK was constructed. The expression vector pSVpTKPA was then constructed by inserting a pPAIV-1-derived, about 18 kb long Sal I fragment containing the 2nd to 14th exons of the TPA gene into said pSVpTK at the Sal I site thereof.

Example 5

Introduction of TPA expression vector into

cultured animal cells and production of TPA

The TPA expression vector plasmids pSVePA-1, pSV3LPA and pSVpTKPA were introduced into various cultured cells following the method of Wigler et al. [Wigler et al. (1977), Cell, vol. 11, p. 223]. In each case, a plasmid-calcium phosphate coprecipitate was added to cells (2 x 10⁵ cells/3.6 ml mdeium/culture dish 6 cm in diameter) grown in advance in Eagle's MEM medium containing 5% fetal calf serum (FCS). After 15 hours of cultivation, the medium was replaced with a fresh portion, and cultivation was continued for 24 hours. The medium was changed for MEM medium containing 5% FCS, 25 µg/ml mycophenolic acid and 250 µg/ml xanthine and cultivation was further continued for 3 weeks. In this way, mycophenolic acid-resistant cell lines were isolated. Mycophenolic acid-resistant cell lines were grown on the whole well bottom surface of a 96-well multidish for 48 hours using MEM medium containing 5% FCS. Then, the activity of TPA contained in the medium in each well was measured using a plasminogen-containing fibrin plate [M. Mackie et al. (1981), British Journal of Haematology, vol. 47, p. 77]. The activity was expressed in terms of units calculated on the urokinase (Green Cross Corp.) activity basis. As shown in Table 1, TPA-producing cell lines were isolated for all the established cell lines used. As regards L929 (mouse, CCL-1), when a TPA gene-free vector, pSVeSall, was introduced thereinto, 10% of mycophenolic acid-resistant cell lines isolated showed an activity of 2 units. As a result of zymography [A. Granelli-Pipern and E. Reich (1978), Journal of Experimental Medicine, vol. 148, p. 223], however, this was distinctly different in molecular weight from human TPA and estimated to be murine plasminogen activating factor [K. R. Marotti et al. (1982), Developmental Biology, vol. 90, p. 154]. In the case of cells with the TPA expression vector introduced thereinto, about 30% of cell lines isolated produced not less than 1 unit of TPA. The estimated molecular weight based on the results of zymography was 65,000, showing that the product was TPA.

Also in CHO-K1 (hamster, CCL-61), Vero (monkey, CCL-81), and WI-26VA4 (human; lung cells transformed with SV40, CCL-95.1), mycophenolic acid-resistant cell lines isolated produced TPA having a molecular weight of 65,000 as estimated by zymography. In CHO-K1 and WI-26VA4, whereas untransfected cells also produced plasminogen activating factor in slight amounts (less than 0.5 unit), transfected cells produced TPA in apparently marked amounts.

Table 1    Production of TPA in different transfected cells

C e l l

| Plasmid introduced | L929 | | CHO-K1 | | Vero | | WI-26VA4 | |
|---|---|---|---|---|---|---|---|---|
| | Isolate* | Activity | Isolate* | Activity | Isolate* | Activity | Isolate* | Activity |
| | LE-22 | 16 | CE- 2 | 48 | VE-38 | 8 | WE- 1 | 48 |
| pSVePA-1 | LE-62 | 48 | CE-22 | 12 | VE-82 | 8 | WE-33 | 32 |
| | LE-105 | 48 | CE-28 | 32 | VE-87 | 12 | WE-67 | 24 |
| | LL- 9 | 48 | CL- 6 | 32 | VL-69 | 24 | WL- 8 | 48 |
| pSV3LPA | LL-15 | 64 | CL-53 | 12 | VL-86 | 8 | WL-11 | 48 |
| | LL-28 | 48 | CL-69 | 24 | VL-124 | 4 | WL-54 | 96 |
| | LT- 4 | 8 | CT-12 | 24 | VT-14 | 2 | WT-25 | 24 |
| pSVpTKPA | LT-32 | 32 | CT-34 | 16 | VT-45 | 2 | WT-106 | 96 |
| | LT-96 | 24 | CT-114 | 16 | VT-67 | 4 | WT-128 | 48 |

* Whereas, for each combination of parent cell line and introduced plasmid, more than 100 mycophenolic acid-resistant cell lines were isolated, the TPA productions by three typical TPA-producing cell lines alone were shown in terms of activity (units) for each of the combinations.

Example 6

Production of TPA in serum-free medium

The mycophenolic acid-resistant cell lines derived from L929, CHO-K1, Vero or WI-26VA4 by transfection with the TPA expression vector pSVePA-1, pSV3LPA or pSVpTKPA as mentioned in Example 5 were each grown in each well of a 24-well multidish plate over whole well bottom surface in MEM medium containing 5% FCS. After washing with serum-free MEM medium, cultivation was continued in the same medium for 48 hours and then the TPA activity contained in the medium was measured using a plasminogen-containing fibrin plate. All the mycophenolic acid-resistant transfectants mentioned in Example 5 produced TPA in amounts equal to to one tenth of the TPA yields attained by cultivation in 5% FCS-containing medium.

Example 7

Introduction of expression vector into

MOPC-31C and MPC-11 and production of TPA

The TPA expression vectors pSVePA-1, pSV3LPA and pSVpTKPA were introduced into the blood cell-derived, established cell lines MOPC-31C (CCL-130) and MPC-11 (CCL-167) by the method of Oi et al. [V. T. Oi et al. (1983), Proc. Natl. Acad. Sci. USA, vol. 80, p. 825]. Thus, Escherichia coli K12 HB101 carrying one of said plasmids was cultivated at 37°C in L medium until the absorbance at 600 nm became 0.6 to 0.8. Chloroamphenicol was added to a concentration of 125 $\mu$g/ml, cultivation was continued further for 12 to 16 hours, and, then, cells were harvested by centrifugation. Cooled 0.05 M Tris-HCl (pH 8.0) containing 20% sucrose was added to 25-ml portions of medium and the bacterial cells were dispersed therein. After addition of 0.25 ml of a lysozyme solution having a concentration of 5 mg/ml, each bacterial suspension was allowed to stand in ice for 5 minutes. Addition of 0.5 ml of 0.05M Tris-HCl (pH 8.0) was followed by incubation at 37°C for 10-15 minutes to thereby convert Escherichia coli to the protoplast. The protoplast was diluted with 10 ml of Dulbecco's modified MEM medium containing 10% sucrose and the dilution was allowed to stand at room temperature for 10 minutes. MOPC-31C or MPC-11 cells were multiplied to $10^6$ cells/ml in Dulbecco's modified MEM medium containing 10% FCS and the protoplast suspension was added thereto in a proportion of 5 ml per 2 x $10^6$ cells, followed by centrifugation at room temperature and about 500 x g. The supernatant was removed and 2 ml of 50% polyethylene glycol solution (pH 8.0) in Dulbecco's modified MEM medium was added. The mixture was stirred gently and then centrifuged at 500 x g for 3 minutes. The cells were washed with 7 ml of Dulbecco's modified MEM medium, then dispersed in Dulbecco's modified MEM medium containing 10% FCS and cultivated on a 24-well multidish at an initial cell concentration of 2 x $10^5$ cells/well·ml.

After 48 hours, the medium was changed for Dulbecco's modified MEM medium containing 10% FCS, 25 $\mu$g/ml mycophenolic acid, 250 $\mu$g/ml xanthine, 5 $\mu$g/ml thymidine, 0.1 $\mu$g/ml aminopterine and 25 $\mu$g/ml adenine, and cultivation was continued for 3-4 weeks. In this way, mycophenolic acid-resistant cell lines were isolated. The mycophenolic acid-resistant cells were cultivated on a 24-well multidish plate over the whole well bottom surface in Dulbecco's modified MEM medium containing 5% FCS for 48 hours. Then, the TPA activity contained in the medium was measured using a plasminogen-containing fibrin plate. As shown in Table 2, transfectant cell lines with any of the expression vectors introduced therein produced TPA.

Table 2  TPA production in blood cell-derived,

established transfectant cell lines

C e l l

| Plasmid intorduced | MOPC-31C | | MPC-11 | |
|---|---|---|---|---|
| | Isolate* | TPA activity | Isolate* | TPA activity |
| | MOE- 3 | 16 | MPE- 2 | 24 |
| pSVePA-1 | MOE- 4 | 16 | MPE-12 | 16 |
| | MOE- 7 | 32 | MPE-14 | 12 |
| | MOL- 9 | 48 | MPL- 1 | 48 |
| pSV3LPA | MOL-16 | 24 | MPL- 2 | 24 |
| | MOL-21 | 48 | MPL- 7 | 24 |
| | MOT- 1 | 24 | MPT- 8 | 48 |
| pSVpTKPA | MOT- 4 | 8 | MPT-11 | 8 |
| | MOT- 7 | 8 | MPT-22 | 12 |

* For each combination of cell line and introduced plasmid, the TPA productions by three typical TPA-rpdocuing mycophenolic acid-resistant cell lines alone are shown in terms of activity (units), although more than 10 such resistant cell lines were isolated.

Example 8

Purification of TPA

The cell lines LE-62 and LE105 derived from L929 by transfection with pSVepA-1 as mentioned in Example 5 were each grown in MEM medium containing 1% FCS to give 300 ml of culture broth. This culture broth, after addition thereto of Tween 80 to a concentration of 0.01%, was subjected to metal chelate affinity chromatography and then to lysine-Sepharose 4B (pharmacia) chromatography, by the method of Porath et al. [J. Porath et al. (1975), Nature, vol. 258, p. 598]. The active fraction thus obtained was dialyzed against 0.05 M Tris-HCl containing 0.1 M sodium chloride and 0.01% Tween 80 to give about 4,000 units of TPA in each case. These TPA products were not neutralized with anti-urokinase antibody but completely neutralized with rabbit antibody to TPA obatined from human melanoma G361 (CRL-1424). Furthermore, upon zymography, these TPA species were in perfect agreement with TPA produced by melanoma G361 with respect to the estimated molecular weight (65,000).

Example 9

Construction of TPA expression vecetor pSVePA-3

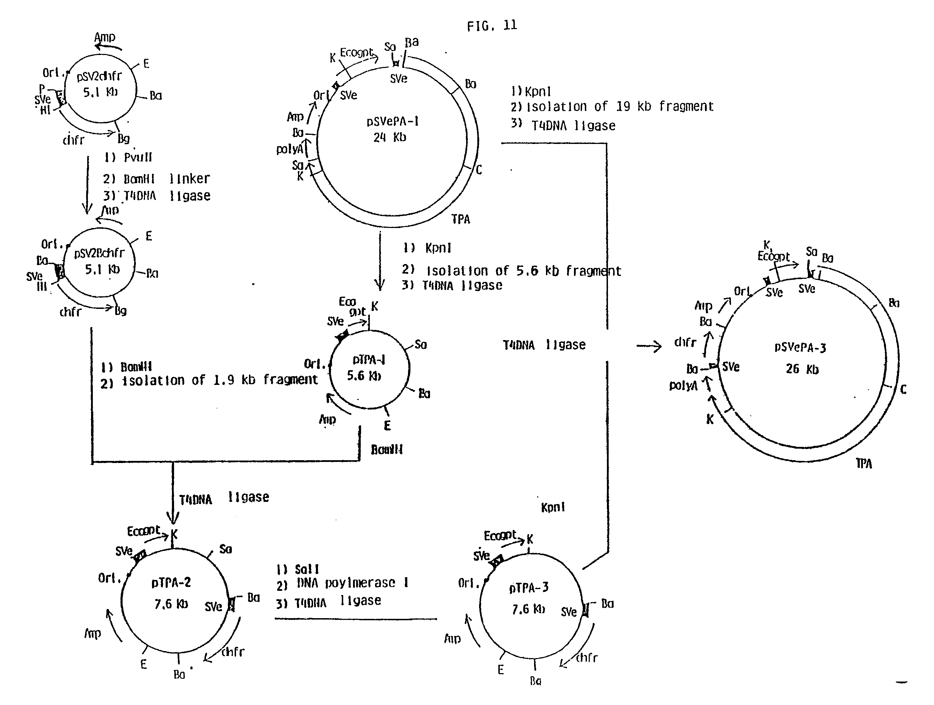

A TPA expression vector having an amplifiable gene, pSVePA-3, was constructed using pSVePA-1 and pSV2dhfr [S. Subramani et al. (1981), Molecular and Cellular Biology, vol. 1, p. 854] as the starting materials and according to the scheme shown in Fig. 11. Thus, pSVePA-1 was cleaved with the restriction enzyme Kpn I and a DNA fragment having the ampicillin resistance gene was made circular to give pTPA-1. Separately, the Pvu II site of pSV2dhfr was converted to a Bam HI site using a Bam HI linker to give a plasmid, pSV2dhfrB. Then, a Bam HI fragment containing the dhfr gene was excised from this plasmid and inserted into pTPA-1 at the Bam HI site thereof to give a plasmid, pTPA2. By erasing the Sal I site of this plasmid, there was constructed a plasmid, pTPA-3. Then, pSVePA-3 was constructed by inserting an about 19 kilobase Kpn I fragment obtained from pSVePA-1 and having the TPA gene into pTPA-3 at the Kpn I site thereof.

Example 10

Transfection of cultured animal cells

with TPA expression vector pSVePA-3

CHO dhfr⁻ [G. Urlaub and L. Chasin (1980), Proc. Natl. Acad. Sci. USA, vol. 77, p. 4216] or BHK-21 (C-13) (ATCC CCL10) was transfected with the TPA expression vector pSVePA-3 or with pSVePA-1 plus pSV2dhfr by the method of Wigler et al. [Wigler et al. (1977), Cell, vol. 11, p. 223]. Thus, a plasmid-calcium phosphate coprecipitate was added to cells (2 x $10^5$ cells/3 ml medium/culture dish 6 cm in diameter) grown in advance in a medium containing 5% fetal calf serum (FCS). After 15 hours, the medium was changed for a fresh one and cultivation was conducted for 48 hours. Then, medium for CHO dhfr⁻ was changed for MEMα medium (GIBCO) containing 5% FCS, 25 μg/ml mycophenolic acid and 250 μg/ml xanthine but free of nucleosides and the medium for BHK for MEM medium containing 5% FCS, 25 μg/ml xanthine, 25 μg/ml adenine, 5 μg/ml thymidine and 0.1 μg/ml aminopterine. Thereafter, cultivation was continued for about 3 weeks. Colonies formed were isolated and grown on a 24-well multiwell plate. After change of the medium for 5% FCS-containing medium, cultivation was carried out for 48 hours and then the TPA activity contained in the medium was determined using a plasminogen-containing fibrin plate [M. Mackie et al. (1981), British Journal of Haematology, vol. 47, p. 77]. The activity was expressed in units calculated on the urokinase (Green Cross Corp.) activity basis. As shown in Table 3, the mycophenolic acid-resistant cell lines produced TPA.

Example 11 Selection of transfected cell lines using methotrexate (Mtx)

Table 3   TPA production by transfected cells*

| Plasmid introduced ($\mu$g/2 x $10^5$ cells/dish) | Host cell | | | |
|---|---|---|---|---|
| | CHOdhfr$^-$ | | BHK-21 (C-13) | |
| | Isolate | Activity | Isolate | Activity |
| pSVePA-3 | C15 | 35 | A07 | 27 |
| (3.5 $\mu$g) | C21 | 37 | A32 | 31 |
| | C69 | 29 | A35 | 22 |
| pSVePA-1 (5.8 $\mu$g) + | D01 | 42 | B29 | 34 |
| pSV2dhfr (1.2 $\mu$g) | D22 | 38 | B41 | 31 |
| | D31 | 39 | B62 | 26 |

* For each cell-plasmid combination, more than 100 mycophenolic acid-resistant cell lines were isolated.  In this table, the TPA productions by three typical TPA-producing cell lines alone are shown in terms of activity (units).

The transfected cell lines derived from CHO dhfr⁻ and BHK-21 (C-13) as obtained in Example 10 were respectively inoculated into dishes 10 cm in diameter in an inoculum size of $10^3$ to $3 \times 10^5$ cells and cultivated in a medium containing 1 nM to 500 nM Mtx, and cells resistant to the respective concentrations of Mtx were isolated. The cells were grown on a 24-well multiwell plate, the medium was changed for a medium containing 5% FCS or free of FCS and, after 48 hours, the TPA activity contained in the medium was measured. As shown in Table 4, cell lines showing higher TPA productivity than the parent cell lines were obtained from among cells selected with Mtx.

Table 4

| Parent cell line | Mtx concentration used for selectrion (nM) | Cell line selected | TPA (units/ml) Culture medium | |
|---|---|---|---|---|
| | | | + FCA | − FCS |
| D01 | 1 | D0102 | 72 | 65 |
| | 20 | D0111 | 115 | 106 |
| | 20 | D0115 | 102 | 88 |
| | 50 | D0138 | 520 | 500 |
| | 50 | D0144 | 380 | 350 |
| | 100 | D0162 | 1610 | 1560 |
| | 100 | D0171 | 1540 | 1460 |
| | 500 | D0191 | 4620 | 4340 |
| C69 | 50 | C6932 | 290 | 220 |
| | 50 | C6933 | 240 | 210 |
| | 500 | C6992 | 1050 | 390 |
| A07 | 125 | A0731 | 310 | 220 |
| | 500 | A0791 | 1340 | 890 |
| B41 | 125 | B4137 | 430 | 390 |
| | 125 | B4139 | 380 | 350 |
| | 250 | B4161 | 1220 | 910 |
| | 250 | B4164 | 1020 | 770 |
| | 500 | B4194 | 3850 | 3100 |
| B62 | 125 | B6233 | 320 | 270 |
| | 125 | B6236 | 290 | 240 |
| | 250 | B6261 | 1080 | 880 |
| | 250 | B6294 | 3120 | 2770 |

**Claims**

1. An expression vector comprising a human chromosomal DNA sequence coding for human tissue plasminogen activating factor, free of the restriction enzime SmaI recognition site between the 8th and the 9th exon and being linked to the promoter region of the thymidine kinase gene promoter of herpes simplex virus, the early promoter of SV40 or the late promoter of SV40.

2. The expression vector according to claim 1, wherein said expression vector contains the T antigen gene of SV40 when the late promoter of SV40 is employed.

3. The expression vector according to claim 1 or 2, which is pSVePA-1, pSV3LPA, pSVpTKPA, or pSVePA-3 having the restriction map given in Figures 8, 9-(b), 10 and 11, respectively.

4. A cell transfected with an expression vector according to any one of claims 1 to 3, which is L929,, CHO-K1, WI-26VA4, MOPC-31C, MPC-11, Vero, CHOdhf⁻ or BH-21 (C-13).

5. The cell according to claim 4, which is capable of being cultured in a serum-free medium.

6. A method of producing a human tissue plasminogen activating factor (TPA) which comprises cultivating a transfected cell according to claim 4 or 5 and isolating the TPA produced thereby.

7. The method according to claim 6, wherein said transfected cell is cultured in a serum-free medium.


**Revendications**

1. Vecteur d'expression comprenant une séquence d'ADN chromosomique humain codant pour le facteur d'activation du plasminogène tissulaire humain, exempt du site de reconnaissance SmaI d'enzyme de restriction entre le huitième et le neuvième exon et qui est liée- à la région promoteur du promoteur du gène de la thymidine kinase du virus herpès simplex, du promoteur précoce de SV40 ou du promoteur tardif de SV40.

2. Vecteur d'expression selon la revendication 1, dans lequel ledit vecteur d'expression contient le gène de l'antigène T de SV40 lorsque l'on utilise le promoteur tardif de SV40.

3. Vecteur d'expression selon la revendication 1 ou 2, qui est pSVePA-1, pSV3LPA, pSVpTKPA, ou pSVePA-3 ayant la carte de restriction donnée dans les figures 8, 9-(b), 10 et 11, respectivement.

4. Cellule transfectée avec un vecteur d'expression selon l'une quelconque des revendications 1 à 3, qui est L929, CHO-KI, WI-26VA4, MOPC-31C, MpC-11, Vero, CHOdhfr⁻ ou BH-21 (C-13).

5. Cellule selon la revendication 4, qui peut être cultivée dans un milieu exempt de sérum.

6. procédé de production du facteur d'activation du plasminogène tissulaire humain (TPA) qui comprend la culture d'une cellule transfectée selon la revendication 4 ou 5 et l'isolement du TPA produit par celle-ci.

7. Procédé selon la revendication 6, dans lequel ladite cellule transfectée est cultivée dans un milieu exempt de sérum.


**Ansprüche**

1. Expressionsvektor, der eine menschliche chromosomale DNA-Sequenz umfaßt, die einen Plasminogen-Aktivierungsfaktor aus menschlichem Gewebe codiert, die keine Erkennungsstelle des Restriktionsenzyms SmaI zwischen dem 8. und 9. Exon enthält, und die mit der Promotorregion des Thymidinkinase-Gen-Promotors des Herpes simplex Virus, dem frühen Promotor von SV40 oder dem späten Promotor von SV40, verknüpft ist.

2. Expressionsvektor nach Anspruch 1, wobei der Expressionsvektor das T-Antigen-Gen von SV40 enthält, wenn der späte Promotor von SV40 verwendet wird.

3. Expressionsvektor nach Anspruch 1 oder 2, der pSVePA-1, pSV3LPA, pSVpTKPA bzw. pSVePA-3 ist und die in der Abbildung 8, 9-(b), 10 bzw. 11 dargestellte Restriktionskarte besitzt.

4. Zelle, die mit einem Expressionsvektor nach einem der Ansprüche 1 bis 3 transfiziert ist und L929, CHO-K1, WI-26VA4, MOPC-31C, MPC-11, Vero, CHOdhfr⁻ oder BH-21 (C-13) ist.

5. Zelle nach Anspruch 4, die in einem serumfreien Medium kultiviert werden kann.

6. Verfahren zur Herstellung eines Plasminogen-Aktivierungsfaktors aus menschlichem Gewebe (TPA), das die Kultivierung einer transfizierten Zelle nach Anspruch 4 oder 5 und die Isolierung des hergestellten TPA umfaßt.

7. Verfahren nach Anspruch 6, wobei die transfizierte Zelle in einem serumfreien Medium kultiviert wird.

F I G. 1

F I G.  2

EP 0 211 260 B1

FIG. 3-(a)

FIG. 3-(b)

EP 0 211 260 B1

FIG. 3(c)

EP 0 211 260 B1

FIG. 3-(e)

FIG. 3-(d)

pPAⅢ
6.8 kb

pPAHin 3·3
5.9 kb

FIG. 4

FIG. 5

FIG. 6

FIG. 7

FIG. 8

FIG. 9-(a)

FIG. 9-(b)

FIG. 10

FIG. 11